# EUROPEAN PATENT SPECIFICATION

(11) **EP 1 383 511 B1**
(45) Date of publication and mention of the grant of the patent: **25.10.2006**
(21) Application number: 01988571.4
(22) Date of filing: 25.10.2001
(51) Int. Cl.: A61K 31/675

(54) **CALCILYTIC COMPOUNDS**
KALZILYTISCHE VERBINDUNGEN
COMPOSES CALCILYTIQUES

(30) Priority: 25.10.2000 US 243007 P
(43) Date of publication of application: 28.01.2004
(73) Proprietor: SMITHKLINE BEECHAM CORPORATION, Philadelphia, PA 19101 (US)
(72) Inventor: BHATNAGAR, Pradip, King of Prussia, PA 19406 (US); BRYAN, William, M., Collegeville, PA 19426 (US); CALLAHAN, James, F., Collegeville, PA 19426 (US); HUFFMAN. William, F., King of Prussia, PA 19406 (US)
(74) Representative: Giddings, Peter John
(86) International application number: PCT/US2001/046233
(87) International publication number: WO 2002/034204

(56) References cited:
- WO-A-00/45816
- WO-A-01/53254
- WO-A-97/37967
- WO-A-98/45255
- WO-A-99/51241
- WO-A-99/51569
- US-A- 5 627 170
- US-A- 5 786 348

## Description

### FIELD OF INVENTION

The present invention relates to novel calcilytic compounds, compositions containing these compounds and their use as calcium receptor antagonists.

In mammals, extracellular Ca²⁺ is under rigid homeostatic control and regulates various processes such as blood clotting, nerve and muscle excitability, and proper bone formation. Extracellular Ca²⁺ inhibits the secretion of parathyroid hormone ("PTH") from parathyroid cells, inhibits bone resorption by osteoclasts, and stimulates secretion of calcitonin from C-cells. Calcium receptor proteins enable certain specialized cells to respond to changes in extracellular Ca²⁺ concentration.

PTH is the principal endocrine factor regulating Ca²⁺ homeostasis in the blood and extracellular fluids. PTH, by acting on bone and kidney cells, increases the level of Ca²⁺ in the blood. This increase in extracellular Ca²⁺ then acts as a negative feedback signal, depressing PTH secretion. The reciprocal relationship between extracellular Ca²⁺ and PTH secretion forms an important mechanism maintaining bodily Ca²⁺ homeostasis.

Extracellular Ca²⁺ acts directly on parathyroid cells to regulate PTH secretion. The existence of a parathyroid cell surface protein which detects changes in extracellular Ca²⁺ has been confirmed. See Brown et al., Nature 366:574, 1993. In parathyroid cells, this protein, the calcium receptor, acts as a receptor for extracellular Ca²⁺, detects changes in the ion concentration of extracellular Ca²⁺ , and initiates a functional cellular response, PTH secretion.

Extracellular Ca²⁺ influences various cell functions, reviewed in Nemeth et al., Cell Calcium 11:319, 1990. For example, extracellular Ca²⁺ plays a role in parafollicular (C-cells) and parathyroid cells. See Nemeth, Cell Calcium 11:323, 1990. The role of extracellular Ca²⁺ on bone osteoclasts has also been studied. See Zaidi, Bioscience Reports 10:493, 1990.

Various compounds are known to mimic the effects of extra-cellular Ca²⁺ on a calcium receptor molecule. Calcilytics are compounds able to inhibit calcium receptor activity, thereby causing a decrease in one or more calcium receptor activities evoked by extracellular Ca²⁺. Calcilytics are useful as lead molecules in the discovery, development, design, modification and/or construction of useful calcium modulators, which are active at Ca²⁺ receptors. Such calcilytics are useful in the treatment of various disease states characterized by abnormal levels of one or more components, e.g., polypeptides such as hormones, enzymes or growth factors, the expression and/or secretion of which is regulated or affected by activity at one or more Ca²⁺ receptors. Target diseases or disorders for calcilytic compounds include diseases involving abnormal bone and mineral homeostasis.

Abnormal calcium homeostasis is characterized by one or more of the following activities: an abnormal increase or decrease in serum calcium; an abnormal increase or decrease in urinary excretion of calcium; an abnormal increase or decrease in bone calcium levels (for example, as assessed by bone mineral density measurements); an abnormal absorption of dietary calcium; an abnormal increase or decrease in the production and/or release of messengers which affect serum calcium levels such as PTH and calcitonin; and an abnormal change in the response elicited by messengers which affect serum calcium levels.

Thus, calcium receptor antagonists offer a unique approach towards the pharmacotherapy of diseases associated with abnormal bone or mineral homeostasis, such as hypoparathyroidism, osteosarcoma, periodontal disease, fracture healing, osteoarthritis, rheumatoid arthritis, Paget's disease, humoral hypercalcemia associated with malignancy and fracture healing, and osteoporosis.

### SUMMARY OF THE INVENTION

The present invention comprises novel calcium receptor antagonists represented by Formula (I) hereinbelow and their use for the preparation of a medicament as calcium receptor antagonists in the treatment of a variety of diseases associated with abnormal bone or mineral homeostasis, including but not limited to hypoparathyroidism, osteosarcoma, periodontal disease, fracture healing, osteoarthritis, rheumatoid arthritis, Paget's disease, humoral hypercalcemia associated with malignancy and fracture healing, and osteoporosis.

The present invention further provides a use for antagonizing calcium receptors in an animal, including humans, which comprises administering to an animal in need thereof an effective amount of a compound of Formula (I), indicated hereinbelow.

The present invention further provides a use for increasing serum parathyroid levels in an animal, including humans, which comprises administering to an animal in need thereof an effective amount of a compound of Formula (I), indicated herein below.

### DETAILED DESCRIPTION OF THE INVENTION

The compounds of the present invention are selected from Formula (I) herein below: 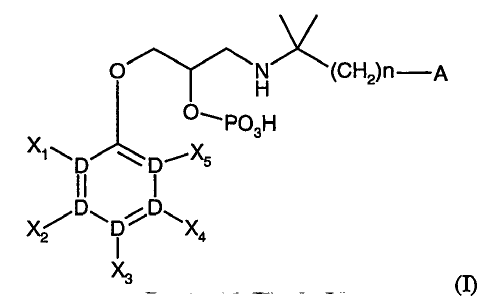 wherein:
A is an aryl or fused aryl, dihydro or tetrahydro fused aryl, heteroaryl or fused heteroaryl, dihydro or tetrahydro fused heteroaryl, unsubstituted or substituted with any substituent being selected from the group consisting of OH, halogen, C₁₋₄alkyl, C₁₋₄akoxy, C₃₋₆ cycloalkyl, CF₃, OCF₃, CN, and NO₂;
D is C or N with up to 2-N in ring, provided that X₁-X₅ are not present when D is N;
X₁ and X₅ are, independently, selected from the group consisting of H, halogen, CN, and NO₂, provided that either X₁ or X₅ is H; further provided that X₁ and X₅ are not present when D is N;
X₂ is selected from the group consisting of H, halogen, O-C₁₋₄ alkyl, and J-K;
X₃ and X₄ are selected from the group consisting of H, halogen, O-C₁₋₄ alkyl, L and J-K;
J is a covalent bond, alkylene, O-alkylene or alkenylene; and
K is selected from the group consisting of, CO₂R₅, CONR₄R'₄, SO₂NR₄R₄, OH, CHO, NR₄R'₄, NR₄SO₂R₄"and CN; provided that X₂, X₃ and X₄ are not present when D is N;
L is 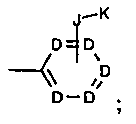
R₄ and R'₄ are independently H, alkyl, aryl or heteroaryl;
R₅ is H, alkyl, alkyl-(O-alkyl)ₘ-O-alkyl, aryl or heteroaryl;
n is an integer from 0 to 4; and,
m is an integer from 1 to 3.

As used herein, "alkyl" refers to an optionally substituted hydrocarbon group joined by single carbon-carbon bonds and having 1-20 carbon atoms joined together. The alkyl hydrocarbon group may be linear, branched or cyclic, saturated or unsaturated. Preferably, substituents on optionally substituted alkyl are selected from the group consisting of aryl, CO₂R, CO₂NHR, OH, OR, CO, NH₂, halo, CF₃, OCF₃ and NO₂, wherein R represents H, C₁₋₄ alkyl, C₃₋₆ cycloalkyl, C₂₋₅ alkenyl, C₂₋₅ alkynyl, heterocycloalkyl, or aryl. Additional substituents are selected from F, Cl, Br, I, N, S and O. Preferably, no more than three substituents are present. More preferably, the alkyl has 1-12 carbon atoms and is unsubstituted. Preferably, the alkyl group is linear.

As used herein "cycloalkyl" refers to optionally substituted 3-7 membered carbocyclic rings wherein any substituents are selected from the group consisting of, F, Cl, Br, I, N(R₄)₂, SR₄ and OR₄, unless otherwise indicated.

As used herein, "aryl" refers to an optionally substituted aromatic group with at least one ring having a conjugated pi-electron system, containing up to two conjugated or fused ring systems. Aryl includes carbocyclic aryl, and biaryl groups, all of which may be optionally substituted. Preferred aryl include phenyl and naphthyl. More preferred aryl include phenyl. Preferred substituents are selected from the group consisting of halogen, C₁₋₄ alkyl, OCF₃, CF₃, OMe, CN, OSO₂ R and NO₂, wherein R represents C₁₋₄ alkyl or C₃₋₆ cycloalkyl.
As used herein, "heteroaryl" refers to an aryl ring containing 1,2 or 3 heteroatoms such as N, S, or O.

As used herein, "alkenyl" refers to an optionally substituted hydrocarbon group containing at least one carbon-carbon double bond and containing up to 5 carbon atoms joined together. The alkenyl hydrocarbon chain may be straight, branched or cyclic. Any substituents are selected from the group consisting of halogen, C₁₋₄ alkyl, OCF₃, CF₃, OMe, CN, OSO₂ R and NO₂, wherein R represents C₁₋₄ alkyl or C₃₋₆ cycloalkyl.

As used herein, "alkynyl" refers to an optionally substituted hydrocarbon group containing at least one carbon-carbon triple bond between the carbon atoms and containing up to 5 carbon atoms joined together. The alkynyl hydrocarbon group may be straight-chained, branched or cyclic. Any substituents are selected from the group consisting of halogen, C₁₋₄ alkyl, OCF₃, CF₃, OMe, CN, OSO₂ R and NO₂, wherein R represents
C₁₋₄ alkyl or C₃₋₆ cycloalkyl.

The compounds of the present invention may contain one or more asymmetric carbon atoms and may exist in racemic and optically active forms. All of these compounds and diastereomers are contemplated to be within the scope of the present invention.

Preferred compounds of the present inventions include:
3-{4-Cyano-3-[(R)-3-(2-indan-2-yl-1,1-dimethyl-ethylamino)-2-phosphonooxy-propoxy]-phenyl}-propionic acid ethyl ester;
3-{4-Cyano-3-[(R)-3-(2-indan-2-yl-1,1-dimethyl-ethylamino)-2-phosphonooxy-propoxy]-phenyl}-propionic acid;
3'-{(R)-3-[2-(5-Chloro-thiophen-2-yl)-1,1-dimethyl-ethylamino]-2-phosphonooxypropoxy}-4'-cyano-biphenyl-4-carboxylic acid ethyl ester; and
3'-{(R)-3-[2-(5-Chloro-thiophen-2-yl)-1,1-dimethyl-ethylamino]-2-phosphonooxypropoxy}-4'-cyano-biphenyl-4-carboxylic acid.

Pharmaceutically acceptable salts are non-toxic salts in the amounts and concentrations at which they are administered.

Pharmaceutically acceptable salts include acid addition salts such as those containing sulfate, hydrochloride, fumarate, maleate, phosphate, sulfamate, acetate, citrate, lactate, tartrate, methanesulfonate, ethanesulfonate, benzenesulfonate, p-toluenesulfonate, cyclohexylsulfamate and quinate. A preferred salt is a hydrochloride. Pharmaceutically acceptable salts can be obtained from acids such as hydrochloric acid, maleic acid, sulfuric acid, phosphoric acid, sulfamic acid, acetic acid, citric acid, lactic acid, tartaric acid, malonic acid, methanesulfonic acid, ethanesulfonic acid, benzenesulfonic acid, p-toluenesulfonic acid, cyclohexylsulfamic acid, fumaric acid, and quinic acid.

Pharmaceutically acceptable salts also include basic addition salts such as those containing benzathine, chloroprocaine, choline, diethanolamine, ethylenediamine, meglumine, procaine, aluminum, calcium, lithium, magnesium, potassium, sodium, ammonium, alkylamine, and zinc, when acidic functional groups, such as carboxylic acid or phenol are present.

The present invention provides compounds of Formula (I) above, which can be prepared using standard techniques. An overall strategy for preparing preferred compounds described herein can be carried out as described in this section. The examples, which follow, illustrate the synthesis of specific compounds. Using the protocols described herein as a model, one of ordinary skill in the art can readily produce other compounds of the present invention.

All reagents and solvents were obtained from commercial vendors. Starting materials were synthesized using standard techniques and procedures.

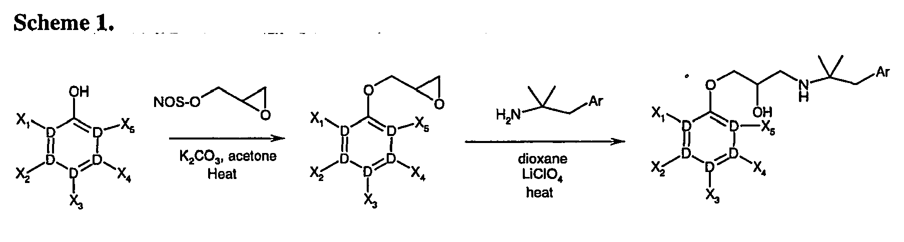

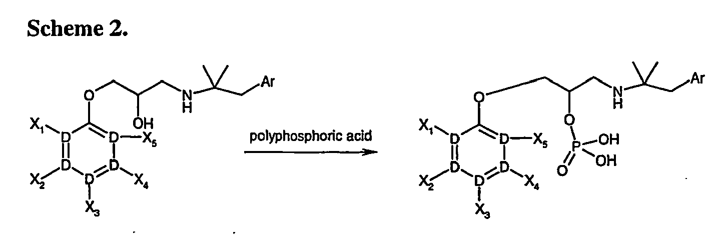

### General Preparation

A general procedure used to synthesize many of the compounds can be carried out as described in Scheme 1, above: a solution of heteroaryl alcohol in acetone was treated with an appropriate base such as K₂CO₃, heated for 15 min. R-glycidyl nosylate was added and the reaction continued overnight to give the corresponding glycidyl ether (Scheme 1). A solution of the substituted glycidyl ether and excess amine (e.g., 1,1-dimethyl-2-(4-methyloxyphenyl)ethylamine) in absolute ethanol, acetonitrile, THF, dioxane, toluene or any other similar solvent in the presence of a suitable catalyst such as LiClO₄ is stirred overnight at reflux. The product is purified by chromatography. Hydrochloride salts are prepared by treatment of the corresponding free base with HCl either in gas phase or 4M dioxane solution, or any other standard method. A representative method to prepare the phosphate esters is shown in Scheme 2.

In order to use a compound of Formula (I) or a pharmaceutically acceptable salt thereof for the treatment of humans and other mammals, it is normally formulated in accordance with standard pharmaceutical practice as a pharmaceutical composition.

The calcilytic compounds can be administered by different routes including intravenous, intraperitoneal, subcutaneous, intramuscular, oral, topical (transdermal), or transmucosal administration. For systemic administration, oral administration is preferred. For oral administration, for example, the compounds can be formulated into conventional oral dosage forms such as capsules, tablets, and liquid preparations such as syrups, elixirs, and concentrated drops.

Alternatively, injection (parenteral administration) may be used, e.g., intramuscular, intravenous, intraperitoneal, and subcutaneous. For injection, the compounds of the invention are formulated in liquid solutions, preferably, in physiologically compatible buffers or solutions, such as saline solution, Hank's solution, or Ringer's solution. In addition, the compounds may be formulated in solid form and redissolved or suspended immediately prior to use. Lyophilized forms can also be produced.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, bile salts and fusidic acid derivatives. In addition, detergents may be used to facilitate permeation. Transmucosal administration, for example, may be through nasal sprays, rectal suppositories, or vaginal suppositories.

For topical administration, the compounds of the invention can be formulated into ointments, salves, gels, or creams, as is generally known in the art.

The amounts of various calcilytic compounds to be administered can be determined by standard procedures taking into account factors such as the compound IC₅₀, EC₅₀, the biological half-life of the compound, the age, size and weight of the patient, and the disease or disorder associated with the patient. The importance of these and other factors to be considered are known to those of ordinary skill in the art.

Amounts administered also depend on the routes of administration and the degree of oral bioavailability. For example, for compounds with low oral bioavailability, relatively higher doses will have to be administered.

Preferably the composition is in unit dosage form. For oral application, for example, a tablet, or capsule may be administered, for nasal application, a metered aerosol dose may be administered, for transdermal application, a topical formulation or patch may be administered and for transmucosal delivery, a buccal patch may be administered. In each case, dosing is such that the patient may administer a single dose.

Each dosage unit for oral administration contains suitably from 0.01 to 500 mg/Kg, and preferably from 0.1 to 50 mg/Kg, of a compound of Formula (I) or a pharmaceutically acceptable salt thereof, calculated as the free base. The daily dosage for parenteral, nasal, oral inhalation, transmucosal or transdermal routes contains suitably from 0.01 mg to 100 mg/Kg, of a compound of Formula (I). A topical formulation contains suitably 0.01 to 5.0% of a compound of Formula (I). The active ingredient may be administered, for example, from 1 to 6 times per day, preferably once, sufficient to exhibit the desired activity, as is readily apparent to one skilled in the art.

As used herein, "treatment" of a disease includes, but is not limited to prevention, retardation and prophylaxis of the disease.

Diseases and disorders which might be treated or prevented, based upon the affected cells, include bone and mineral-related diseases or disorders; hypoparathyroidism; those of the central nervous system such as seizures, stroke, head trauma, spinal cord injury, hypoxia-induced nerve cell damage, such as occurs in cardiac arrest or neonatal distress, epilepsy, neurodegenerative diseases such as Alzheimer's disease, Huntington's disease and Parkinson's disease, dementia, muscle tension, depression, anxiety, panic disorder, obsessive-compulsive disorder, post-traumatic stress disorder, schizophrenia, neuroleptic malignant syndrome, and Tourette's syndrome; diseases involving excess water reabsorption by the kidney, such as syndrome of inappropriate ADH secretion (SIADH), cirrhosis, congestive heart failure, and nephrosis; hypertension; preventing and/or decreasing renal toxicity from cationic antibiotics (e.g., aminoglycoside antibiotics); gut motility disorders such as diarrhea and spastic colon; GI ulcer diseases; GI diseases with excessive calcium absorption such as sarcoidosis; autoimmune diseases and organ transplant rejection; squamous cell carcinoma; and pancreatitis.

In a preferred embodiment of the present invention, the present compounds are used to increase serum parathyroid hormone ("PTH") levels. Increasing serum PTH levels can be helpful in treating diseases such as hypoparathyroidism, osteosarcoma, periodontal disease, fracture, osteoarthritis, rheumatoid arthritis, Paget's disease, humoral hypercalcemia malignancy and osteoporosis.

In a preferred embodiment of the present invention, the present compounds are co-administered with an anti-resorptive agent. Such agents include, but are not limited estrogen, 1, 25 (OH)₂ vitamin D3, calcitonin, selective estrogen receptor modulators, vitronectin receptor antagonists, V-H+-ATPase inhibitors, src SH2 antagonists, bisphosphonates and cathepsin K inhibitors.

Another aspect of the present invention describes a method of treating a patient comprising administering to the patient an amount of a present compound sufficient to increase the serum PTH level. Preferably, the method is carried out by administering an amount of the compound effective to cause an increase in duration and/or quantity of serum PTH level sufficient to have a therapeutic effect.

In various embodiments, the compound administered to a patient causes an increase in serum PTH having a duration of up to one hour, about one to about twenty-four hours, about one to about twelve hours, about one to about six hours, about one to about five hours, about one to about four hours, about two to about five hours, about two to about four hours, or about three to about six hours.

In an alternative embodiment of the present invention, the compound administered to a patient causes an increase in serum PTH having a duration of more than about twenty four hours provided that it is co-administered with an anti resorptive agent.

In additional different embodiments, the compound administered to a patient causes an increase in serum PTH of up to two fold, two to five fold, five to ten fold, and at least 10 fold, greater than peak serum PTH in the patient. The peak serum level is measured with respect to a patient not undergoing treatment.

Composition of Formula (I) and their pharmaceutically acceptable salts, which are active when given orally, can be formulated as syrups, tablets, capsules and lozenges. A syrup formulation will generally consist of a suspension or solution of the compound or salt in a liquid carrier for example, ethanol, peanut oil, olive oil, glycerine or water with a flavoring or coloring agent. Where the composition is in the form of a tablet, any pharmaceutical carrier routinely used for preparing solid formulations may be used. Examples of such carriers include magnesium stearate, terra alba, talc, gelatin, acacia, stearic acid, starch, lactose and sucrose. Where the composition is in the form of a capsule, any routine encapsulation is suitable, for example using the aforementioned carriers in a hard gelatin capsule shell. Where the composition is in the form of a soft gelatin shell capsule any pharmaceutical carrier routinely used for preparing dispersions or suspensions may be considered, for example aqueous gums, celluloses, silicates or oils, and are incorporated in a soft gelatin capsule shell.

Typical parenteral compositions consist of a solution or suspension of a compound or salt in a sterile aqueous or non-aqueous carrier optionally containing parenterally acceptable oil, for example polyethylene glycol, polyvinylpyrrolidone, lecithin, arachis oil or sesame oil.

Typical compositions for inhalation are in the form of a solution, suspension or emulsion that may be administered as a dry powder or in the form of an aerosol using a conventional propellant such as dichlorodifluoromethane or trichlorofluoromethane.

A typical suppository formulation comprises a compound of Formula (I) or a pharmaceutically acceptable salt thereof which is active when administered in this way, with a binding and/or lubricating agent, for example polymeric glycols, gelatins, cocoa-butter or other low melting vegetable waxes or fats or their synthetic analogs.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

No unacceptable toxological effects are expected when compounds of the present invention are administered in accordance with the present invention.

The biological activity of the compounds of Formula (I) are demonstrated by the following tests:

### (I) Calcium Receptor Inhibitor Assay

Calcilytic activity was measured by determining the IC₅₀ of the test compound for blocking increases of intracellular Ca²⁺ elicited by extracellular Ca²⁺ in HEK 293 4.0-7 cells stably expressing the human calcium receptor. HEK 293 4.0-7 cells were constructed as described by Rogers et al., J. Bone Miner. Res. 10 Suppl. 1:S483, 1995 (hereby incorporated by reference herein). Intracellular Ca²⁺ increases were elicited by increasing extracellular Ca²⁺ from 1 to 1.75 mM. Intracellular Ca²⁺ was measured using fluo-3, a fluorescent calcium indicator.

The procedure was as follows:
1. Cells were maintained in T-150 flasks in selection media (DMEM supplemented with 10% fetal bovine serum and 200 ug/mL hygromycin B), under 5% CO₂:95% air at 37 °C and were grown up to 90% confluency.
2. The medium was decanted and the cell monolayer was washed twice with phosphate-buffered saline (PBS) kept at 37 °C. After the second wash, 6 mL of 0.02% EDTA in PBS was added and incubated for 4 minutes at 37 °C. Following the incubation, cells were dispersed by gentle agitation.
3. Cells from 2 or 3 flasks were pooled and pelleted (100 x g). The cellular pellet was resuspended in 10-15 mL of SPF-PCB+ and pelleted again by centrifugation. This washing was done twice.
   Sulfate- and phosphate-free parathyroid cell buffer (SPF-PCB) contains 20 mM Na-Hepes, pH 7.4, 126 mM NaCl, 5 mM KCl, and 1 mM MgCl₂. SPF-PCB was made up and stored at 4 °C. On the day of use, SPF-PCB was supplemented with 1 mg/mL of D-glucose and 1 mM CaCl₂ and then split into two fractions. To one fraction, bovine serum albumin (BSA; fraction V, ICN) was added at 5 mg/mL (SPF-PCB+). This buffer was used for washing, loading and maintaining the cells. The BSA-free fraction was used for diluting the cells in the cuvette for measurements of fluorescence.
4. The pellet was resuspended in 10 mL of SPF-PCB+ containing 2.2 uM fluo-3 (Molecular Probes) and incubated at room temperature for 35 minutes.
5. Following the incubation period, the cells were pelleted by centrifugation. The resulting pellet was washed with SPF-PCB+. After this washing, cells were resuspended in SPF-PCB+ at a density of 1-2 x 106 cells/mL.
6. For recording fluorescent signals, 300 uL of cell suspension were diluted in 1.2 mL of SPF buffer containing 1 mM CaCl₂ and 1 mg/mL of D-glucose. Measurements of fluorescence were performed at 37 °C with-constant stirring using a spectrofluorimeter. Excitation and emission wavelengths were measured at 485 and 535 nm, respectively. To calibrate fluorescence signals, digitonin (5 mg/mL in ethanol) was added to obtain Fmax, and the apparent Fmin was determined by adding Tris-EGTA (2.5 M Tris-Base, 0.3 M EGTA). The concentration of intracellular calcium was calculated using the following equation: Intracellular calcium = (F-Fₘᵢₙ/Fₘₐₓ) x K_{d}; where K_{d} = 400 nM.
7. To determine the potential calcilytic activity of test compounds, cells were incubated with test compound (or vehicle as a control) for 90 seconds before increasing the concentration of extracellular Ca²⁺ from 1 to 2mM. Calcilytic compounds were detected by their ability to block, in a concentration-dependent manner, increases in the concentration of intracellular Ca²⁺ elicited by extracellular Ca²⁺.

In general, those compounds having lower IC₅₀ values in the Calcium Receptor Inhibitor Assay are more preferred compounds. Compounds having an IC₅₀ greater than 50 uM were considered to be inactive. Preferred compounds are those having an IC₅₀ of 10uM or lower, more preferred compounds have an IC₅₀ of 1uM, and most preferred compounds have an IC₅₀ of 0.1uM or lower.

### (II) Calcium Receptor Binding Assay

HEK 293 4.0-7 cells stably transfected with the Human Parathyroid Calcium Receptor ("HuPCaR") were scaled up in T180 tissue culture flasks. Plasma membrane is obtained by polytron homogenization or glass douncing in buffer (50mM Tris-HCl pH 7.4, 1mM EDTA, 3mM MgCl₂) in the presence of a protease inhibitor cocktail containing 1uM Leupeptin, 0.04 uM Pepstatin, and 1 mM PMSF. Aliquoted membrane was snap frozen and stored at -80 °C. ³H labeled compound was radiolabeled to a radiospecific activity of 44Ci/mmole and was aliquoted and stored in liquid nitrogen for radiochemical stability.

A typical reaction mixture contains 2 nM ³H compound ((R,R)-N-4'-Methoxy-t-3-3'-methyl-1'-ethylphenyl-1-(1-naphthyl)ethylamine), or ³H compound (R)-N-[2-Hydroxy-3-(3-chloro-2-cyanophenoxy)propyl]-1,1-dimethyl-2-(4-methoxyphenyl)ethylamine 4-10 ug membrane in homogenization buffer containing 0.1% gelatin and 10% EtOH in a reaction volume of 0.5 mL. Incubation is performed in 12 x 75 polyethylene tubes in an ice water bath. To each tube 25 uL of test sample in 100% EtOH is added, followed by 400 uL of cold incubation buffer, and 25 uL of 40 nM ³H-compound in 100% EtOH for a final concentration of 2nM. The binding reaction is initiated by the addition of 50 uL of 80-200 ug/mL HEK 293 4.0-7 membrane diluted in incubation buffer, and allowed to incubate at 4°C for 30 min. Wash buffer is 50 mM Tris-HCl containing 0.1 % PEI. Nonspecific binding is determined by the addition of 100-fold excess of unlabeled homologous ligand, and is generally 20% of total binding. The binding reaction is terminated by rapid filtration onto 1% PEI pretreated GF/C filters using a Brandel Harvestor. Filters are placed in scintillation fluid and radioactivity assessed by liquid scintillation counting.

### Examples

Nuclear magnetic resonance spectra were recorded at either 250 or 400 MHz using, respectively, a Bruker AM 250 or Bruker AC 400 spectrometer. CDCl₃ is deuteriochloroform, DMSO-d₆ is hexadeuteriodimethylsulfoxide, and CD₃OD is tetradeuteriomethanol. Chemical shifts are reported in parts per million (•) downfield from the internal standard tetramethylsilane. Abbreviations for NMR data are as follows: s=singlet, d=doublet, t=triplet, q=quartet, m=multiplet, dd=doublet of doublets, dt=doublet of triplets, app=apparent, br=broad. J indicates the NMR coupling constant measured in Hertz. Continuous wave infrared (IR) spectra were recorded on a Perkin-Elmer 683 infrared spectrometer, and Fourier transform infrared (FTIR) spectra were recorded on a Nicolet Impact 400 D infrared spectrometer. IR and FTIR spectra were recorded in transmission mode, and band positions are reported in inverse wavenumbers (cm⁻¹). Mass spectra were taken on either VG 70 FE, PE Syx API III, or VG ZAB HF instruments, using fast atom bombardment (FAB) or electrospray (ES) ionization techniques. Elemental analyses were obtained using a Perkin-Elmer 240C elemental analyzer. Melting points were taken on a Thomas-Hoover melting point apparatus and are uncorrected. All temperatures are reported in degrees Celsius.

Analtech Silica Gel GF and E. Merck Silica Gel 60 F-254 thin layer plates were used for thin layer chromatography. Both flash and gravity chromatographs were carried out on E. Merck Kieselgel 60 (230-400-mesh) silica gel. Analytical and preparative HPLC were carried out on Rainin or Gilson chromatographs. ODS refers to an octadecylsilyl derivatized silica gel chromatographic support. 5 µ Apex-ODS indicates an octadecylsilyl derivatized silica gel chromatographic support having a nominal particle size of 5 µ, made by Jones Chromatography, Littleton, Colorado. YMC ODS-AQ® is an ODS chromatographic support and is a registered trademark of YMC Co. Ltd., Kyoto, Japan. PRP-1® is a polymeric (styrenedivinylbenzene) chromatographic support, and is a registered trademark of Hamilton Co., Reno, Nevada) Celite® is a filter aid composed of acid-washed diatomaceous silica, and is a registered trademark of Manville Corp., Denver, Colorado. Following the general procedure described above the following compounds have been synthesized:

### Example 1

### Preparation of 2-Indan-2-yl-1,1-dimethyl-ethylamine

**a) Indan-2-yl-acetic acid methyl ester**
   A solution of indan-2-yl-acetic acid (Lancaster, 20 g, 0.11 mol) in methanol (200 mL) was stirred and cooled to 0-10 °C in an ice bath and treated drop-wise with thionyl chloride (14.8 g, 0.125 mol). The mixture was stirred at RT for 16 h, concentrated *in vacuo,* and the oily residue was dissolved in ethyl acetate, washed with 2.5 N sodium hydroxide, water, and brine, dried (MgSO₄), and concentrated *in vacuo* to give the title compound (21 g, 97%) which solidified.
**b) 1-Indan-2-yl-2-methyl-propan-2-ol** A solution of the compound from Example 1(a) (6.3 g, 33 mmol) in ether (150 mL) was added drop-wise to 1.4 M methyllithium in ether (100 mL, 4.25 eq) stirred in an ice bath. The mixture was allowed to warm to RT, stirred for 2 h, and very carefully quenched by drop-wise addition of saturated aqueous ammonium chloride (150 mL). The aqueous phase was separated and extracted with ether, and the combined ether phase was washed with brine, dried (MgSO₄), and concentrated *in vacuo* to afford the title compound as an oil which crystallized on standing (~89%).
**c) N-(2-Indan-2-yl-1,1-dimethyl-ethyl)-acetamide**
   To a mixture of concentrated sulfuric acid (1.7 mL) in acetonitrile (6 mL) stirred in an ice bath for 45 min. a drop-wise solution of the compound from Example 1(b) (3.3 g, 17.3 mmol) in glacial acetic acid (5 mL) was added. The mixture was allowed to warm to RT, stirred for 16 h, poured into ice water, and extracted with ethyl acetate. The combined organic extract was washed with 2.5 N sodium hydroxide, water, and brine, dried (MgSO₄), and concentrated *in vacuo* to give an oily residue that was triturated with hexane and a few drops of ethyl acetate, seeded, and cooled to afford a solid which was isolated by filtration to afford the title compound as tan solid (1.9 g, 47%). MS(ES) m/e 231.9 [M+H]⁺. The filtrate was concentrated *in vacuo* to afford additional title compound as an oil (1.5 g, 37%).
**d) 2-Indan-2-yl-1,1-dimethyl-ethylamine**
   A mixture of the compound from Example 1(c) (6.5 g, 28 mmol) in ethylene glycol (170 mL) was treated with crushed potassium hydroxide pellets (13 g), stirred, and heated to 190°C for 24 h. The mixture was poured into water and extracted with ethyl acetate. The combined organic phase was washed with brine and extracted with 1 N hydrochloric acid. The combined acidic extract was washed with ethyl acetate, basified with 2.5 N sodium hydroxide, and extracted with ethyl acetate. The combined organic extract was washed with brine, dried (MgSO₄), and concentrated *in vacuo* to afford the title compound (3.2 g, 60%). MS(ES) m/e 190.6 [M+H]⁺.

### Example 2

### Preparation of ethyl (R)-4-cyano-3-(oxiranylmethoxy)benzenepropionate

**a) Ethyl 3-hydroxybenzenepropionate**
   A solution of 3-(3-hydroxyphenyl)propionic acid (Lancaster, 66.4 g, 0.4 mol) in ethanol (700 mL) was treated with concentrated sulfuric acid (6 mL), heated to reflux for 2 h, and allowed to cool to RT. The mixture was cooled in ice, neutralized with 10% aqueous sodium carbonate and concentrated *in vacuo* to about 50 mL. Water (-200 mL) was added and the mixture was extracted three-times with ethyl acetate. The combined ethyl acetate extract was washed with water and brine, dried (Na₂SO₄), filtered, and concentrated *in vacuo* to yield the title compound as an oil (70 g, 90%).
**b) Ethyl 4-formyl-3-hydroxybenzenepropionate** To a solution of the compound from Example 2(a) (77.2 g, 0.4 mol) in dry acetonitrile (1L) stirred under argon was added triethylamine (152 g, 1.5 mol) followed by magnesium chloride (57.1 g, 0.6 mol). After stirring for 5 min, paraformaldehyde (81 g) was added and the reaction was refluxed under argon for 1.5 h. The reaction was cooled, 6 N hydrochloric acid (400 mL) was added and the resulting mixture was extracted with ethyl acetate. The combined ethyl acetate extract was washed with water, dried (MgSO₄), filtered and concentrated *in vacuo.* The residual oil was purified by flash column chromatography (silica gel, 10% ethyl acetate/hexane) to give the title compound (66.6 g, 75%).
**c) Ethyl 3-hydroxy-4-[(hydroxyimino)methyl]benzenepropionate** A solution of the compound from Example 2(b) (66.6 g, 0.3 mol) in absolute ethanol (500 mL) was treated with triethylamine (40.4 g, 0.4 mol) followed by hydroxylamine hydrochloride (23 g, 0.33 mol). The reaction was stirred under argon at reflux for 18 h, concentrated *in vacuo,* and the residual oil was dissolved in ethyl acetate and washed with 1N hydrochloric acid. The ethyl acetate phase was dried (MgSO₄), filtered, and concentrated *in vacuo* to give the title compound as an oil which was used in the next step.
**d) Ethyl 3-acetoxy-4-cyanobenzenepropionate** The compound from Example 2(c) was treated with acetic anhydride (500 mL) and refluxed under argon for 90 min. The reaction was concentrated *in vacuo* and the resulting oil was dissolved in ethyl acetate and washed with water. The ethyl acetate layer was dried (MgSO₄), filtered, and concentrated *in vacuo* to give the title compound as an oil which was used in the next step.
**e) Ethyl 4-cyano-3-hydroxybenzenepropionate** A solution of the compound from Example 2(d) was dissolved in ethanol (200 mL) and treated with a solution of sodium carbonate (64 g, 0.6 mol) in water (1.5 L). After stirring at RT for 5 h, the mixture was neutralized with 6 N hydrochloric acid to pH 5 and concentrated *in vacuo.* The resulting mixture was extracted with ethyl acetate. The ethyl acetate solution was dried (MgSO₄), filtered, and concentrated *in vacuo* to give the title compound as an oil [61.9 g, 94.2% overall yield from the compound of Preparation 2(c)].
**f) Ethyl (R)-4-cyano-3-(oxiranylmethoxy)benzenepropionate** A solution of the compound from Example 2(e) (28.3 g, 0.13 mol) and (2R)-glycidyl 3-nitrobenzenesulfonate (33.7 g, 0.13 mol) in dry acetone (500 mL) was treated with potassium carbonate (36 g, 0.26 mol) and refluxed under argon for 18 h. The reaction was cooled, filtered, and the filtrate was concentrated *in vacuo* and the residue was purified by flash column chromatography (silica, 30% ethyl acetate/hexane) to yield the title compound (29.5 g, 82.4%).

### Example 3

### Preparation of Ethyl 3-{4-Cyano-3-[(R)-2-hydroxy-3-(2-indan-2-yl-1,1-dimethyl-ethylamino)-propoxy]-phenyl}-propionate

A mixture the compounds from Example 2(f) (6 g, 31.7 mmol) and Example 1(d) (8.6 g, 31.7 mmol) in absolute ethanol (200 mL) was stirred and heated to reflux for 56 h, cooled, concentrated *in vacuo.* The residue was dissolved in dichloromethane (30 mL) and acidified with 1.0 N hydrogen chloride in ether. The white solid which formed was isolated by filtration and recrystallized to afford the title compound (10 g, 63%). mp (dichloromethane/ether) 155-157°C; MS(ES) m/e 465.4 [M+H]⁺.

### Example 4

### Preparation of 3-{4-Cyano-3-[(R)-3-(2-indan-2-yl-1,1-dimethyl-ethylamino)-2-phosphonooxy-propoxy]-phenyl}-propionic acid ethyl ester and 3-{4-Cyano-3-[(R)-3-(2-indan-2-yl-1,1-dimethyl-ethylamino)-2-phosphonooxy-propoxy]-phenyl}-propionic acid

The compound from Example 3 (0.2 g, 0.4 mmol) in polyphosphoric acid (3 g) was allowed to stand at RT for 2 days. The mixture was diluted with water (2 mL), dimethylsulfoxide (2 mL) and ethanol (2 mL). This solution (2 mL) was subjected to preperative HPLC on a 50 X 20 mm ID YMC Combiprep ODS column eluting at 20 mL/min with a linear gradient of 20% MeCN(0.1% TFA)/80% water(0.1% TFA) to 60%MeCN(0.1% TFA) over 15 min to give the ester (20 mg); MS(ES) m/z: 545.2 [M+H]⁺ and the acid (6 mg); MS(ES) m/z: 517.2 [M+H]⁺.

### Example 5

### Preparation of 4'-Cyano-3'-((R)-1-oxiranylmethoxy)-biphenyl-4-carboxylic acid ethyl ester

### a) 2-Hydroxyl-4-bromobenzonitrile

A mixture of 2-fluoro-5-bromobenzonitrile (30g, 152.3 mmole), potassium acetate (222.4 g, 228.5 mmol), and 18-crown-6 ether (60.4g, 288.5 mmol) in MeCN (400 mL) was heated at reflux in 36h. The mixture was cooled, 2.5 N NaOH (200mL) added, and stirred at RT overnight. The mixture was extracted with ether (discarded). The aqueous layer was acidified with 6N HCl, extracted with EtOAc, dried over MgSO₄, concentrated, purified by flash column chromatography (40% EtOAc/Hex) to give the above titled compound as a light yellow foam (24.45g, 81%). NMR(300MHz, DMSO-d₆): □□7.13(dd, J=1.7, 8.3Hz, 1H), 7.17(d, J=1.7Hz, 1H), 7.61(d, J=8.3Hz, 1H).

### b) Ethyl-4-[[3-hydroxyl-4-cyano]phenyl]benzoate

A mixture of Example 5(a) (3.0 g, 1.52 mmol), p-carboxylbenzene boronic acid (3.02g, 1.82 mmol), (Ph₃P)₄Pd (0.88 g, 0.76 mmol), and 2M Na₂CO₃ (38mL, 7.6mmole) in toluene/EtOH (60 mL, 4:1) was heated at reflux in 24h. The mixture was cooled, and the layers were separated. The aqueous layer was extracted with ether (discarded), acidified with 6N HCl. The tan solid (3.6g, 99%) was filtered, dried, dissolved in EtOH, and added 4M HCl in p-dioxane (20mL). The resulting mixture was heated at reflux in 24h. The mixture was cooled, concentrated, taken up in H₂O, stirred, filtered, and dried to give the abovetitled compound as an off white solid (3.75g, 85%). ¹H-NMR (300MHz, DMSO-d₆): δ 1.32 (t, J=7.1Hz, 3H), 4.34 (q, J=7.1Hz, 2H), 7.29 (s,1H), 7.31 (d, J=8.3Hz, 1H), 7.73 (d, J=8.3Hz, 1H), 7.76 (d, J=8.3Hz, 2H), 8.06 (d, J=8.3Hz, 2H).

### c) Ethyl-4-[[3-[[R-glycidyl]oxyl]methyl-4-cyano]phenyl]benzoate

A mixture of Example 5(b) (0.71g, 2.7 mmol), potassium carbonate (0.73 g, 5.3 mmol), and R-glycidyl-3-nitrobenzenesulfonate (0.73 g, 2.8 mmol) in acetone (30 mL) was heated at relux in 24h. The mixture was cooled, concentrated, taken up in H₂O, extracted with EtOAc. The organic extracts were washed with brine, dried over MgSO₄, concentrated to afford the above titled compound as an off white solid (0.7g, 82%). NMR (300MHz, DMSO-d₆): δ□ 1.32(t, J=7.1Hz, 3H), 2.78(dd, J=2.6, 4.9Hz, 1H), 2.91(t, J=4.9, 1H), 3.44(m, 1H), 4.20(dd, J=6.5, 11.7, 1H), 4.39(q, J=7.1Hz, 2H), 4.72(dd, J=2.6, 11.7Hz, 1H), 7.47(dd, J=1.4, 8Hz, 1H), 7.57(d, J=1.4Hz, 1H), 7.85(d, J=8Hz, 1H), 7.95(d, J=8.5Hz, 1H), 8.08(d, J=8.5Hz, 1H).

### Example 6

### Preparation of 2-(5-Chloro-thiophen-2-yl)-1,1-dimethyl-ethylamine

### a) 3-(5-Chloro-thiophen-2-yl)-2,2-dimethyl-propionic acid methyl ester

To a cooled (-78 °C) stirred solution of diisopropylamine (9.54g, 94.3 mmol) in anhydrous THF (100 mL) was added 37.8 mL of a 2.5M solution of n-BuLi under N₂. After stirring at (-78 °C for 30 min., methyl isobutyrate (9.2g, 89.8 mmol) was added, and continued to stir for 1h. A solution o 5-chloro-2-methylchlorothiophene (15 g, 89.8 mmol) in dried THF (50 mL) was added. The reaction mixture was allowed to stir for an additional 4h at that temperature, it was then allowed to warm to RT, quenched with water, extracted with ether (3x100 mL). The combined organic extracts were washed with brine, dried over Mg SO₄, filtered, and concentrated to afford the above titled compound as a brown oil (19.2, 93%). ¹H -NMR (300MHz, CDCl₃) δ 1.21 (s, 6H), 2.95 (s, 2H), 3.7 (s, 3H), 6.52 (d, J= 3.6 Hz, 1H), 6.71 (d, J= 3.6 Hz, 1H).

### b) 3-(5-Chloro-thiophen-2-yl)-2,2-dimethyl-propionic acid

A mixture of compound from Example 6(a) (19.5 g, 83.8 mmol) and 2.5 N NaOH (67 mL, 167.7 mmol) in p-dioxane (200 mL) was heated at 80 °C for 24h. The mixture was then cooled, the organic solvent eliminated in vacuo, the aqueous residue was extracted with ether (discarded). The aqueous layer was acidified with 6N HCl to pH=4, extracted with CH₂Cl₂ (3x75 mL). The combined organic extracts were washed with brine, dried over Mg SO₄, filtered, and concentrated to afford the above titled compound as a brown oil (16.5,90%). ¹H -NMR (300MHz, CDCl₃) δ 1.25 (s, 6H), 2.98 (s, 2H), 6.60 (d, J=3.6 Hz, 1H), 6.75 (d, J=3.6 Hz, 1H).

### c) 2-Chloro-5-(2-isocyanato-2-methyl-propyl)-thiophene

To a stirred mixture of compound from Example 6(b) (16.5g, 75.5 mmol), and triethyl amine (8.41 g, 83.1 mmol) in dried toluene (200 mL) was added diphenylphosphoryl azide (22.6 g, 83.1 mmol). The reaction mixture was stirred at room temperature for 1 h, then heated at 80 °C for 2h, cooled to room temperature, quenched with water and the layers were separated. The aqueous layer was extracted with ether (3x 100 mL). The combined organic extracts were washed with brine, dried over Mg SO₄, filtered, and concentrated to afford the above titled compound as a brown oil (16.3, 100%). ¹H - NMR (300MHz, CDCl₃) δ 1.38 (s, 6H), 2.88 (s, 2H), 6.65 (d, J=3.6 Hz, 1H), 6.79 (d, J=3.6 Hz, 1H).

### d) 2-(5-Chloro-thiophen-2-yl)-1,1-dimethyl-ethylamine

To a stirred solution of compound from example 6(c) (16.3g, 75.5 mmol) in diglyme (200 mL), 6N HCl was added (200 mL). The reaction mixture was heated at 120 °C for 2h, cooled, concentrated under high vacuum, and taken up in water, extracted with ether (discarded). The acidic aqueous layer was brought to pH= 8 with a 50% NaOH solution, then extracted with CH₂Cl₂ (5x50 mL). The combined organic extracts were washed with brine, dried over Mg SO₄, filtered, and concentrated to afford the above titled compound as a brown oil (12.5, 87%). ¹H NMR (300MHz, DMSO-d₆) δ 1.01 (s, 6H), 2.71 (s, 2H), 6.70 (d, J=3.6 Hz, 1H), 6.92 (d, J=3.6 Hz, 1H).

### Example 7

### Preparation of 3'-{(R)-3-[2-(5-Chloro-thiophen-2-yl)-1,1-dimethyl-ethylamino]-2-hydroxy-propoxy}-4'-cyano-biphenyl-4-carboxylic acid ethyl ester

The solution of compounds from Example 5(c) (5 g, 15.47 mmol) and 6(d) (4.39 g, 23 mmol) in dioxane/CH₃CN (1:1, 250 mL) were treated with LiClO₄ (1.7 g, 16.1 mmol) and heated at reflux for 3 d. The reaction mixture was cooled and evaporated. The residue was purified by flash chromatography to give the above titled compound (6.23 g, 78.6%). MS(ES) m/z 513 [M+H]⁺.

### Example 8

### Preparation of 3'-{(R)-3-[2-(5-Chloro-thiophen-2-yl)-1,1-dimethyl-ethylamino]-2-phosphonooxy-propoxy}-4'-cyano-biphenyl-4-carboxylic acid ethyl ester

The compound from Example 7 (0.5 g, 0.98 mmol) in polyphosphoric acid (5 g) was allowed to stand at RT for 4 days. The mixture was diluted with water (50 mL) and the solid was filtered. The solid (200 mg) was subjected to preperative HPLC on a 50 X 20 mm ID YMC Combiprep ODS column eluting at 20 mL/min with a linear gradient of 40% MeCN(0.1% TFA)/60% water(0.1% TFA) to 60%MeCN(0.1% TFA) over 15 min to give the above titled compound (50 mg). MS(ES) m/z: 593 [M+H]⁺.

### Example 9

### Preparation of 3'-{(R)-3-[2-(5-Chloro-thiophen-2-yl)-1,1-dimethyl-ethylamino]-2-phosphonooxy-propoxy}-4'-cyano-biphenyl-4-carboxylic acid

The crude solid from Example 8 (60 mg) in 1N sodium hydroxide (1mL) and ethanol (0.5 mL) was stirred at RT for 3 days. The mixture was concentrated to 1 mL and subjected to preperative HPLC on a 50 X 20 mm ID YMC Combiprep ODS column eluting at 20 mL/min with a linear gradient of 20% MeCN(0.1% TFA)/80% water(0.1% TFA) to 60%MeCN(0.1% TFA) over 15 min to give the above titled compound (30 mg). MS(ES) m/z: 565.2 [M+H]⁺.

## Claims

1. A compound according to formula (I) hereinbelow:
or a pharmaceutically acceptable salt thereof. 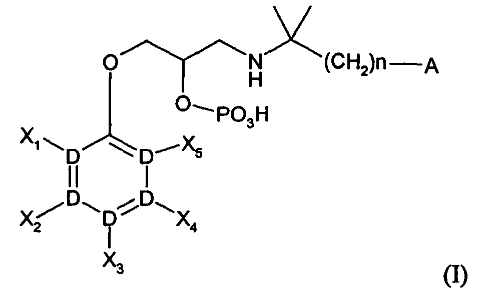
wherein:
A is an aryl or fused aryl, dihydro or tetrahydro fused aryl, heteroaryl or fused heteroaryl, dihydro or tetrahydro fused heteroaryl, unsubstituted or substituted with any substituent being selected from the group consisting of OH, halogen, C₁₋₄alkyl, C₁₋₄alkoxy, C₃₋₆ cycloalkyl, CF₃, OCF₃, CN, and NO₂;
D is C or N with up to 2-N in ring, provided that X₁-X₅ are not present when D is N;
X₁ and X₅ are, independently, selected from the group consisting of H, halogen, CN, and NO₂, provided that either X₁ or X₅ is H; further provided that X₁ and X₅ are not present when D is N;
X₂ is selected from the group consisting ofH, halogen, O-C₁₋₄ alkyl, and J-K; X₃ and X₄ are selected from the group consisting of H, halogen, O-C₁₋₄ alkyl, L and J-K;
J is a covalent bond, alkylene, O-alkylene or alkenylene; and
K is selected from the group consisting of, CO₂R₅, CONR₄R'₄, SO₂NR₄R₄, OH, CHO, NR₄R'₄, NR₄SO₂R₄"and CN; provided that X₂, X₃ and X₄ are not present when D is N;
L is 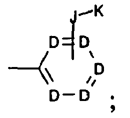
R₄ and R'₄ are independently H, alkyl, aryl or heteroaryl;
R₅ is H, alkyl, alkyl-(O-alkyl)ₘ-O-alkyl, aryl or heteroaryl;
n is an integer from 0 to 4; and,
m is an integer from 1 to 3.

2. A compound according to claim 1 selected from the group consisting of: 3- {4-Cyano-3-[(R)-3-(2-indan-2-yl-1,1-dimethyl-ethylamino)-2-phosphonooxypropoxy]-phenyl}-propionic acid ethyl ester;
3- {4-Cyano-3-[(R)-3-(2-indan-2-yl-1,1-dimethyl-ethylamino)-2-phosphonooxypropoxy]-phenyl}-propionic acid;
3'- {(R)-3-[2-(5-Chloro-thiophen-2-yl)-1,1-dimethyl-ethylamino]-2-phosphonooxy-propoxy}-4'-cyano-biphenyl-4-carboxylic acid ethyl ester; and
3'- {(R)-3-[2-(5-Chloro-thiophen-2-yl)-1,1-dimethyl-ethylamino]-2-phosphonooxy-propoxy}-4'-cyano-biphenyl-4-carboxylic acid.

3. The use of a compound as claimed in claim 1 or claim 2 in the manufacture of a medicament for antagonizing a calcium receptor.

4. The use of a compound as claimed in claim 1 or claim 2 in the manufacture of a medicament for treating a disease or disorder **characterized by** an abnormal bone or mineral homeostasis.

5. A use according to claim 4 wherein the bone or mineral disease or disorder is selected from the group consisting of osteosarcoma, periodontal disease, fracture healing, osteoarthritis, joint replacement, rheumatoid arthritis, Paget's disease, humoral hypercalcemia, malignancy and osteoporosis.

6. A use according to claim 5 wherein the bone or mineral disease or disorder is osteoporosis.

7. The use of a compound as claimed in claim 1 or claim 2 in the manufacture of a medicament for increasing serum parathyroid levels

8. A use according to claim 6 or claim 7 wherein the compound is co-administered with an anti-resorptive agent.

9. A use according to claim 8 wherein the anti-resorptive agent is selected from the group consisting of: estrogen, 1, 25 (OH)₂ vitamin D3, calcitonin, selective estrogen receptor modulators, vitronectin receptor antagonists, V-H+-ATPase inhibitors, src SH2 antagonists, bisphosphonates and cathepsin K inhibitors.

10. A pharmaceutical composition comprising a compound according to Claim 1 or claim2 and a pharmaceutically acceptable carrier or diluent

11. A method of synthesizing a compound according to claim 1 or claim 2 by phosphorylating the corresponding aryloxypropinolamines selectively at the secondary alcohol with polyphosphoric acid.

## Patentansprüche

1. Verbindung gemäß Formel (I) unten:
oder ein pharmazeutisch annehmbares Salz davon, 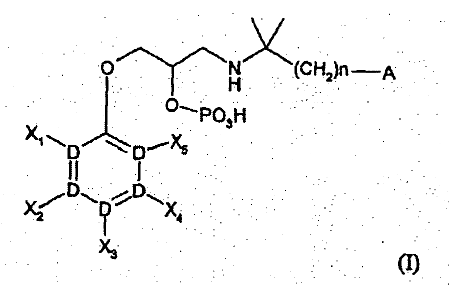 worin A ein Aryl oder fusioniertes Aryl, Dihydro oder Tetrahydro-fusioniertes Aryl, Heteroaryl oder fusioniertes Heteroaryl, Dihydro oder Tetrahydro-fusioniertes Heteroaryl, nicht-substituiert oder substituiert mit irgendeinem Substituenten, der aus der Gruppe ausgewählt ist, die aus OH, Halogen, C₁₋₄-Alkyl, C₁₋₄-Alkoxy, C₃₋₆-Cycloalkyl, CF₃, OCF₃, CN und NO₂ besteht;
D C oder N mit bis zu 2-N im Ring ist, mit der Maßgabe, dass X₁-X₅ nicht vorhanden sind, wenn D N ist;
X₁ und X₅ unabhängig voneinander ausgewählt sind aus der Gruppe, bestehend aus H, Halogen, CN, und NO₂ mit der Maßgabe, dass entweder X₁ oder X₅ H ist; mit der weiteren Maßgabe, dass X₁ und X₅ nicht vorhanden sind, wenn D N ist;
X₂ ist aus der Gruppe ausgewählt, die aus H, Halogen, O-C₁₋₄-Alkyl und J-K besteht;
X₃ und X₄ werden aus der Gruppe ausgewählt, die aus H, Halogen, O-C₁₋₄-Alkyl, L und J-K ausgewählt wird;
J ist eine kovalente Bindung, Alkylen, O-Alkylen oder Alkenylen; und
K wird aus der Gruppe ausgewählt, die aus CO₂R₅, CONR₄R'₄, SO₂NR₄R₄, OH, CHO, NR₄R'₄, NR₄SO₂R₄" und CN besteht; mit der Maßgabe, dass X₂, X₃ und X₄ nicht vorhanden sind, wenn D N ist;
L ist 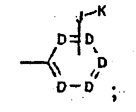
R₄ und R'₄ sind unabhängig H, Alkyl, Aryl oder Heteroaryl;
R₅ ist H, Alkyl, Alkyl-(O-alkyl)ₘ-O-alkyl, Aryl oder Heteroaryl;
n ist eine ganze Zahl von 0 bis 4; und
m ist eine ganze Zahl von 1 bis 3.

2. Verbindung gemäß Anspruch 1, ausgewählt aus der Gruppe, bestehend aus 3-{4-Cyano-3-[(R)-3-(2-indan-2-yl-1-1-dimethyl-ethylamino)-2-phosphonoxy-propyl]-phenyl}-propionsäureethylester;
3-{4-Cyano-3-[(R)-3-(2-indan-2-yl-1,1-dimethyl-ethylamino)-2-phosphonoxy-propoxy]-phenyl}-propionsäure;
3'-{(R)-3-[2-(5-Chlorthiophen-2-yl)-1,1-dimethyl-ethylamino]-2-phosphonoxy-propoxy}-4'-cyano-biphenyl-4-carbonsäureethylester; und
3'-{(R)-3-[2-(5-Chlor-thiophenyl-2-yl)-1,1-dimethyl-ethylamino]-2-phosphonoxy-propoxy}-4'-cyano-biphenyl-4-carbonsäure.

3. Verwendung einer Verbindung gemäß Anspruch 1 oder 2 zur Herstellung eines Medikaments zur Antagonisierung eines Calciumrezeptors.

4. Verwendung einer Verbindung wie in Anspruch 1 oder Anspruch 2 beansprucht, zur Herstellung eines Medikaments zur Behandlung einer Erkrankung oder Störung, die durch abnormale Knochen- oder Mineralhomöostase gekennzeichnet ist.

5. Verwendung gemäß Anspruch 4, wobei die Knochen- oder Mineralerkrankung oder -störung, ausgewählt aus der Gruppe, bestehend aus Osteosarkom, Periodontalerkrankung, Bruchheilung, Osteoarthritis, Gelenkersatz, rheumatoide Arthritis, Pagets-Erkrankung, humorale Hyperkalzämie, Malignität und Osteoporose.

6. Verwendung gemäß Anspruch 5, wobei die Knochen- oder Mineralerkrankung oder Störung Osteoporose ist.

7. Verwendung einer Verbindung gemäß Anspruch 1 oder 2 zur Herstellung eines Medikaments zur Erhöhung der Konzentration von Serum-Nebenschilddrüsenhormon.

8. Verwendung gemäß Anspruch 6 oder 7, worin die Verbindung gemeinsam mit einem anti-resorptiven Mittel verabreicht wird.

9. Verwendung gemäß Anspruch 8, worin das anti-resorptive Mittel ausgewählt ist aus der Gruppe, bestehend aus: Östrogen, 1,25 (OH)₂ Vitamin D₃, Calcitonin, selektiven Östrogenrezeptormodulatoren, Vitronectinrezeptorantagonisten, V-H⁺-ATPase-Inhibitoren, Src-SH2-Antagonisten, Bisphosphonaten und Cathepsin K-Inhibitoren.

10. Pharmazeutische Zusammensetzung, umfassend eine Verbindung gemäß Anspruch 1 oder 2, und einen pharmazeutisch annehmbaren Träger oder Verdünnungsmittel.

11. Verfahren zum Synthetisieren einer Verbindung gemäß Anspruch 1 oder 2 durch Phosphorylieren der entsprechenden Aryloxypropionolamine mit Polyphosphorsäure selektiv am zweiten Alkohol.

## Revendications

1. Composé selon la formule (I) ci-dessous, ou sel pharmaceutiquement acceptable de celui-ci : 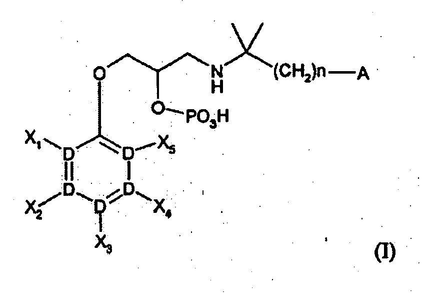 dans lequel :
A est un aryle ou un aryle fusionné, un dihydro- ou un tétrahydro- aryle fusionné, un hétéroaryle ou un hétéroaryle fusionné, un dihydro- ou un tétrahydro-hétéroaryle fusionné, non substitué ou substitué par un quelconque substituant choisi dans le groupe constitué de OH, d'un halogène, d'un alkyle en C₁ à C₄, d'un alcoxy en C₁ à C₄, d'un cycloalkyle en C₃ à C₆, de CF₃, de OCF₃, de CN, et de NO₂ ;
D est C ou N avec jusqu'à 2 N dans le cycle, à condition que X₁ à X₅ ne soient pas présents lorsque D est N ;
X₁ et X₅ sont indépendamment choisis dans le groupe constitué de H, d'un halogène, de CN, et de NO₂, à condition que soit X₁ soit X₅ soit H ; à condition encore que X₁ et X₅ ne soient pas présents lorsque D est N ;
X₂ est choisi dans le groupe constitué de H, d'un halogène, d'un O-(alkyle en C₁ à C₄), et de J-K ;
X₃ et X₄ sont choisis dans le groupe constitué de H, d'un halogène, d'un O-(alkyle en C₁ à C₄), de L et de J-K ;
J est une liaison covalente, un alkylène, un O-alkylène ou un alcénylène ; et
K est choisi dans le groupe constitué de CO₂R₅, de CONR₄R'₄, de SO₂NR₄R₄, de OH, de CHO, de NR₄R'₄, de NR₄SO₂R₄" et de CN ; à condition que X₂, X₃ et X₄ ne soient pas présents lorsque D est N ;
L est 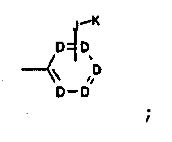
R₄ et R'₄ sont indépendamment H, un alkyle, un aryle ou un hétéroaryle ;
R₅ est H, un alkyle, un alkyl-(O-alkyle)ₘ-O-alkyle, un aryle ou un hétéroaryle ;
n est un entier de 0 à 4 ; et
m est un entier de 1 à 3.

2. Composé selon la revendication 1, choisi dans le groupe constitué de :
l'ester éthylique de l'acide 3-{4-cyano-3-[(R)-3-(2-indan-2-yl-1,1-diméthyl-éthylamino)-2-phosphonooxypropoxy]phényle} propionique ;
l'acide 3-{4-cyano-3-[(R)-3-(2-indan-2-yl-1,1-diméthyl-éthylamino)-2-phosphonooxy-propoxy] phényle} propionique ;
l'ester éthylique de l'acide 3'-{(R)-3-[2-(5-chloro-thiophèn-2-yle)-1,1-diméthyléthylamino]-2-phosphonooxy-propoxy}-4'-cyano-biphényl-4 -carboxylique ; et
l'acide 3'-{(R)-3-[2-(5-chloro-thiophèn-2-yle)-1,1 -diméthyl-éthylamino]-2-phosphonooxy-propoxy}-4'-cyano-biphényl-4-carboxylique.

3. Utilisation d'un composé selon la revendication 1 ou la revendication 2, dans la fabrication d'un médicament pour l'antagonisation d'un récepteur du calcium.

4. Utilisation d'un composé selon la revendication 1 ou la revendication 2, dans la fabrication d'un médicament pour le traitement d'une maladie ou d'un trouble **caractérisé par** une anomalie osseuse ou une homéostasie minérale.

5. Utilisation selon la revendication 4, dans laquelle la maladie ou le trouble osseux ou minéral est choisi dans le groupe constitué de l'ostéosarcome, de la maladie périodontale, de la cicatrisation d'une fracture, l'ostéoarthrite, du remplacement d'une articulation, de l'arthrite rhumatoïde, de la maladie de Paget, de l'hypercalcémie humorale, d'une malignité et de l'ostéoporose.

6. Utilisation selon la revendication 5, dans laquelle la maladie ou le trouble osseux ou minéral est l'ostéoporose.

7. Utilisation d'un composé selon la revendication 1 ou la revendication 2, dans la fabrication d'un médicament pour augmenter les niveaux de l'hormone parathyroïdienne dans le sérum.

8. Utilisation selon la revendication 6 ou la revendication 7, dans laquelle le composé est co-administré avec un agent anti-résorption.

9. Utilisation selon la revendication 8, dans laquelle l'agent anti-résorption est choisi dans le groupe constitué de : l'oestrogène, la vitamine D3 1, 25 (OH)₂, la calcitonine, les modulateurs sélectifs du récepteur de l'oestrogène, les antagonistes du récepteur de la vitronectine, les inhibiteurs de la V-H+-ATPase, les antagonistes de src SH2, les bisphosphonates et les inhibiteurs de la cathepsine K.

10. Composition pharmaceutique comprenant un composé selon la revendication 1 ou la revendication 2 et un support ou un diluant pharmaceutiquement acceptable.

11. Procédé de synthétisation d'un composé selon la revendication 1 ou la revendication 2, par phosphorylation sélective des aryloxypropinolamines correspondantes au niveau de l'alcool secondaire avec de l'acide polyphosphorique.
